# DEMANDE DE BREVET EUROPEEN

(11) **EP 1 424 063 A1**
(43) Date de publication de la demande: **02.06.2004**
(21) Numéro de dépôt: 03292532.3
(22) Date de dépôt: 13.10.2003
(51) Int. Cl.: A61K 7/42, C07D 403/04, C08K 5/34

(54) **Compositions cosmétiques photoprotectrices contenant des dérivés de 3-(2-azacycloalkylidène)-1,3-dihydro-indol-2-one utilisations**

(30) Priorité: 29.11.2002 FR 0215054
(71) Demandeur: L'OREAL, 75008 Paris (FR)
(72) Inventeur: Rozot, Roger, 77400 Lagny/Marne (FR)
(74) Mandataire: Miszputen, Laurent

(57) **Abrégé**

L'invention concerne des compositions cosmétiques ou dermatologiques, destinées à la photoprotection de la peau et/ou des cheveux contre le rayonnement UV, en particulier le rayonnement solaire comprenant une quantité efficace d'au moins un dérivé de 3-(2-azacycloalkylidene)-1,3-dihydro-indol-2-one de structure particulière.

L'invention concerne également l'utilisation dans la préparation de formulations cosmétiques ou dermatologiques photoprotectrices de ces dérivés de 3-(2-azacycloalkylidene)-1,3-dihydro-indol-2-one particuliers à titre de filtres solaires actifs dans le domaine des rayonnements UV.

## Description

L'invention concerne des compositions cosmétiques ou dermatologiques, destinées à la photoprotection de la peau et/ou des cheveux contre le rayonnement UV, en particulier le rayonnement solaire comprenant une quantité efficace d'au moins un dérivé de 3-(2-azacycloalkylidene)-1,3-dihydro-indol-2-one de structure particulière.

L'invention concerne également l'utilisation dans la préparation de formulations cosmétiques ou dermatologiques photoprotectrices de ces dérivés de 3-(2-azacycloalkylidene)-1,3-dihydro-indol-2-one particuliers à titre de filtres solaires actifs dans le domaine des rayonnements UV.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande croissante de moyens de contrôle de ce bronzage naturel afin de contrôler ainsi la couleur de leur peau. Il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreux composés destinés à la photoprotection (UV-A et/ou UV-B) de la peau ont été proposés à ce jour.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire s'exprimant mathématiquement par le rapport du temps d'irradiation nécessaire pour atteindre le seuil érythématogène avec le filtre UV au temps nécessaire pour atteindre le seuil érythématogène sans filtre UV).

Outre leur pouvoir filtrant, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne stabilité vis à vis des radiations UV (photostabilité).

La Demanderesse a découvert de manière surprenante une nouvelle famille de filtres UV organiques du type dérivé de 3-(2-azacycloalkylidene)-1,3-dihydro-indol-2-one dépourvus d'activité photo-oxydante et présentant, outre des propriétés filtrantes excellentes dans le domaine des rayonnements UV-A et/ou UV-B, une très bonne solubilité dans les solvants organiques usuels et notamment les corps gras tels que les huiles, une excellente photostabilité ainsi que d'excellentes propriétés cosmétiques, les rendant particulièrement appropriés à une utilisation comme filtres solaires dans des compositions cosmétiques destinées à la protection de la peau et/ou des cheveux contre le rayonnement ultraviolet.

La présente invention a ainsi pour premier objet une composition cosmétique à usage topique, en particulier pour la photoprotection de la peau et/ou des cheveux, caractérisée par le fait qu'elle comprend, dans un support cosmétiquement acceptable au moins un dérivé de 3-(2-azacycloalkylidene)-1,3-dihydro-indol-2-one répondant à la formule (1) suivante : 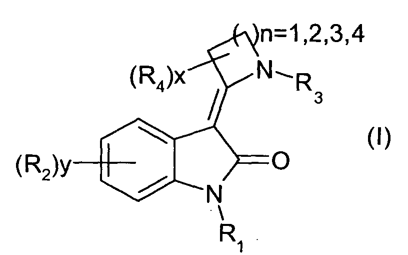 dans laquelle :
**R**_{**1**} **et R**_{**3**} sont identiques ou différents et peuvent être :
   - un atome d'hydrogène,
   - un groupe alkyle en C₁-C₂₂ linéaire ou ramifié, saturé ou insaturé, éventuellement substitué par un ou plusieurs groupes A₁,
   - un cycle de 4 à 7 atomes, saturé ou insaturé, contenant éventuellement au moins un hétéroatome parmi N, O et S, éventuellement accolé à un autre cycle, éventuellement substitué par un ou plusieurs groupes A₁, ces cycles pouvant comporter une ou plusieurs fonctions carbonyle ou thiocarbonyle,
   - un des groupes C(=NR₅)R'₅, C(=NR₅)NR'₅R"₅, COR₅, CSR₅, COOR₅, CONR₅R'₅, CSNR₅R'₅, SO₂R₅, SO₂NR₅R'₅ avec R₅, R'₅ et R"₅ identiques ou différents désignant l'hydrogène, un radical alkyle en C₁-C₂₂, linéaire ou ramifié ou un cycle de 4 à 7 atomes, pouvant contenir au moins un hétéroatome parmi N, O, S, isolé ou accolé à un autre cycle, ces cycles pouvant comporter une ou plusieurs fonctions carbonyle ou thiocarbonyle, le radical alkyle ou lesdits cycles étant saturés ou non et éventuellement substitués par au moins un substituant A₂.
**R**_{**2**} **et R**_{**4**} sont identiques ou différents et peuvent être :
   - un atome d'hydrogène,
   - un groupe alkyle en C₁-C₂₂ linéaire ou ramifié, saturé ou insaturé éventuellement, substitué par un ou plusieurs groupes A₁,
   - un cycle de 4 à 7 atomes, saturé ou insaturé, contenant éventuellement au moins un hétéroatome parmi N, O et S, éventuellement substitué par un ou plusieurs groupes A₁, ce cycle pouvant accolé à un autre cycle ou aux cycles de la structure 3-(2-azacycloalkylidene)-1,3-dihydro-indol-2-one objet de l'invention, ces cycles pouvant comporter une ou plusieurs fonctions carbonyle ou thiocarbonyle, ce cycle pouvant également être chargé positivement comme imidazolium, pyridinium, pyrazolium, triazolium,
   - un halogène comme F, Cl, Br,
   - un des groupes CF₃, CN, OR₅, SR₅, NR₅R'₅, C(=NR₅)R'₅, C(=NR₅)NR'₅R"₅, NR₅C(=NR'₅)NR"₅R"'₅, COR₅, CSR₅, COOR₅, CONR₅R'₅, NR₅COR'₅, NR₅CONR'₅R"₅, CSNR₅R'₅, SO₂NR₅R'₅, NR₅SO₂R'₅, SO₂R₅, NR₅R'₅R"₅R"'₅⁺ avec R₅, R'₅, R"₅ et R"'₅ identiques ou différents désignant l'hydrogène, un radical alkyle en C₁-C₂₂, linéaire ou ramifié ou un cycle de 4 à 7 atomes, pouvant contenir au moins un hétéroatome parmi N, O, S, isolé ou accolé à un autre cycle, ces cycles pouvant comporter une ou plusieurs fonctions carbonyle ou thiocarbonyle, le radical alkyle ou lesdits cycles étant saturés ou non et éventuellement substitués par au moins un substituant A₂ ;
**A**_{**1**} peut être :
   - un halogène comme F, Cl, Br,
   - un groupe alkyle en C₁C₂₂ linéaire ou ramifié, saturé ou insaturé, éventuellement substitué par un ou plusieurs groupes A₂,
   - un des groupes CF₃, CN, OR₅, SR₅, NR₅R'₅, C(=NR₅)R'₅, C(=NR₅)NR'₅R"₅, NR₅C(=NR'₅)NR"₅R"'₅, COR₅, CSR₅, COOR₅, CONR₅R'₅, NR₅COR'₅, NR₅CONR'₅R"₅, CSNR₅R'₅, SO₂NR₅R'₅, NR₅SO₂R'₅, SO₂R₅, SiR₅R'₅R"₅, SiR₅(OSiR'₅R"₅ R"'₅)OSiR'₅R"₅R"'₅, NR₅R'₅R"₅R"'₅⁺ avec R₅, R'₅, R"₅ et R"'₅ identiques ou différents désignant l'hydrogène, un radical alkyle en C₁-C₂₂, linéaire ou ramifié ou un cycle de 4 à 7 atomes, pouvant contenir au moins un hétéroatome parmi N, O, S, isolé ou accolé à un autre cycle, ces cycles pouvant comporter une ou plusieurs fonctions carbonyle ou thiocarbonyle, le radical alkyle ou lesdits cycles étant saturés ou non et éventuellement substitués par au moins un substituant A₂
   - un cycle de 4 à 7 atomes, saturé ou insaturé, contenant éventuellement au moins un hétéroatome parmi N, O et S, éventuellement substitué par un ou plusieurs groupes A₂, ce cycle pouvant accolé à un autre cycle ou aux cycles de la structure 3-(2-azacycloalkylidene)-1,3-dihydro-indol-2-one objet de l'invention, ces cycles pouvant comporter une ou plusieurs fonctions carbonyle ou thiocarbonyle, ce cycle pouvant également être chargé positivement comme imidazolium, pyridinium, pyrazolium, triazolium,
**A**_{**2**} peut être :
   - un halogène comme F, Cl, Br,
   - un groupe alkyle en C₁-C₂₂ linéaire ou ramifié, saturé ou insaturé,
   - un des groupes CF₃, CN, OR, SR, NRR', C(=NR)R', C(=NR)NR'R", NRC(=NR')NR"R"', COR, CSR, COOR, CONRR', NRCOR', NRCONR'R", CSNRR', SO₂NRR' NRSO₂R', SO₂R, SiRR'R", SiR(OSiR'R"R"')OSiR'R"R"', NRR'R"R"'⁺ avec R, R', R" et R"' identiques ou différents désignant l'hydrogène ou un radical alkyle en C₁-C₂₂, linéaire ou ramifié.
y vaut 1,2,3 ou 4 ;
x vaut de 1 à 2n+2.

Comme exemples de groupes alkyle en C₁-C₂₂ linéaires ou ramifiés, saturés ou insaturés, on peut citer: méthyle, isopropyle, éthyle-2-hexyle, *ter*-butyle, éthylène, propylène. Cette liste n'est pas limitative.

Comme exemples d'hétérocycles, on peut citer : pyrrole, furanne, thiophène, imidazole, oxazole, thiazole, pyrazole, isoxazole, isothiazole, triazole, oxadiazole, thiadiazole, tétrazole, pyridine, pipéridine, pyrimidine, pipérazine, pyridazine, pyrazine, triazine, morpholine, pyrrolidine, thiazolidine. Cette liste n'est pas limitative.

Comme exemples de cycles carbonés à 4, 5, 6 ou 7 atomes, saturés ou insaturés, on peut citer: cyclobutyle, cyclopentyle, cyclohexyle, cyclohexényle, phényle ou cycloheptyle. Cette liste n'est pas limitative.

Certains dérivés de 3-(2-azacycloalkylidene)-1,3-dihydro-indol-2-one de formule (1) conformes à l'invention sont connus dans la littérature chimique et notamment certains d'entre eux ont été décrits dans les articles de *Indian Journal of Chemistry* (1983), 22B, 1083 - 1086 et de *Journal of Medicinal Chemistry* (1989), 32, 437 - 444.

Les dérivés de 3-(2-azacycloalkylidene)-1,3-dihydro-indol-2-one de formule (1) conformes à l'invention peuvent être préparés selon un procédé de synthèse mettant en oeuvre un acétal de lactame et un dérivé de 1,3-dihydro-indol-2-one à température ambiante dans l'éther anhydre selon le schéma réactionnel suivant cité dans les articles ci-dessus :

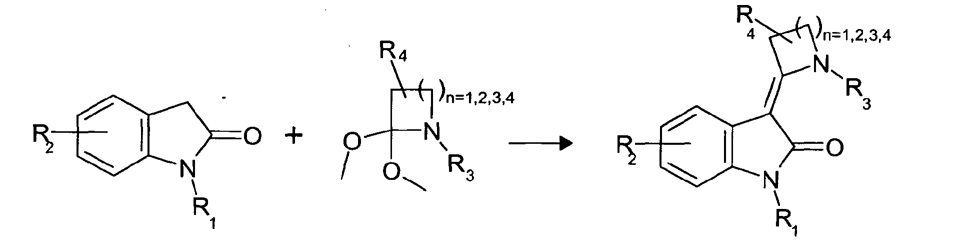

De nombreux dérivés lactame et 1,3-dihydro-indol-2-one sont disponibles chez la plupart dés fournisseurs de produits chimiques comme par exemple :

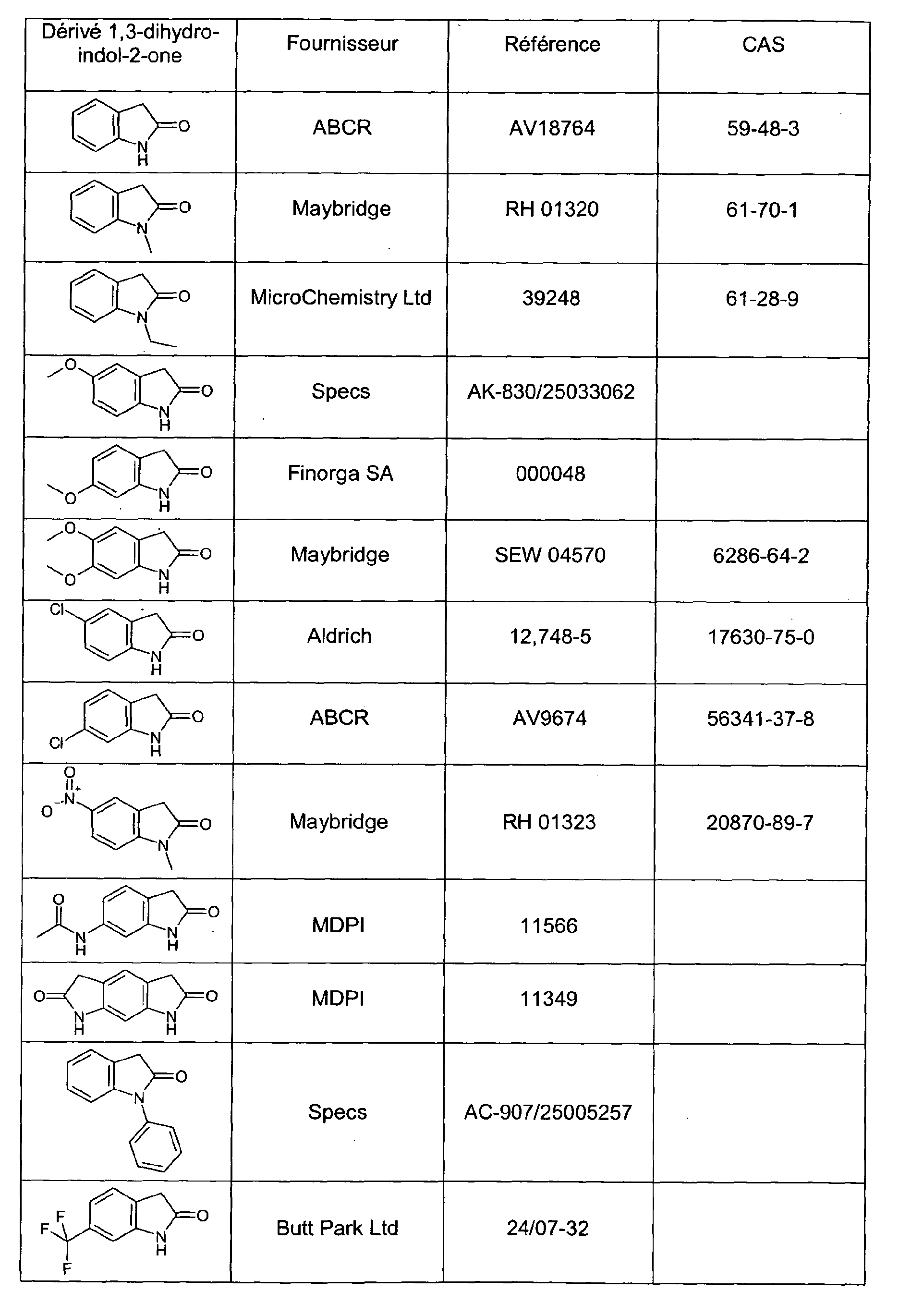

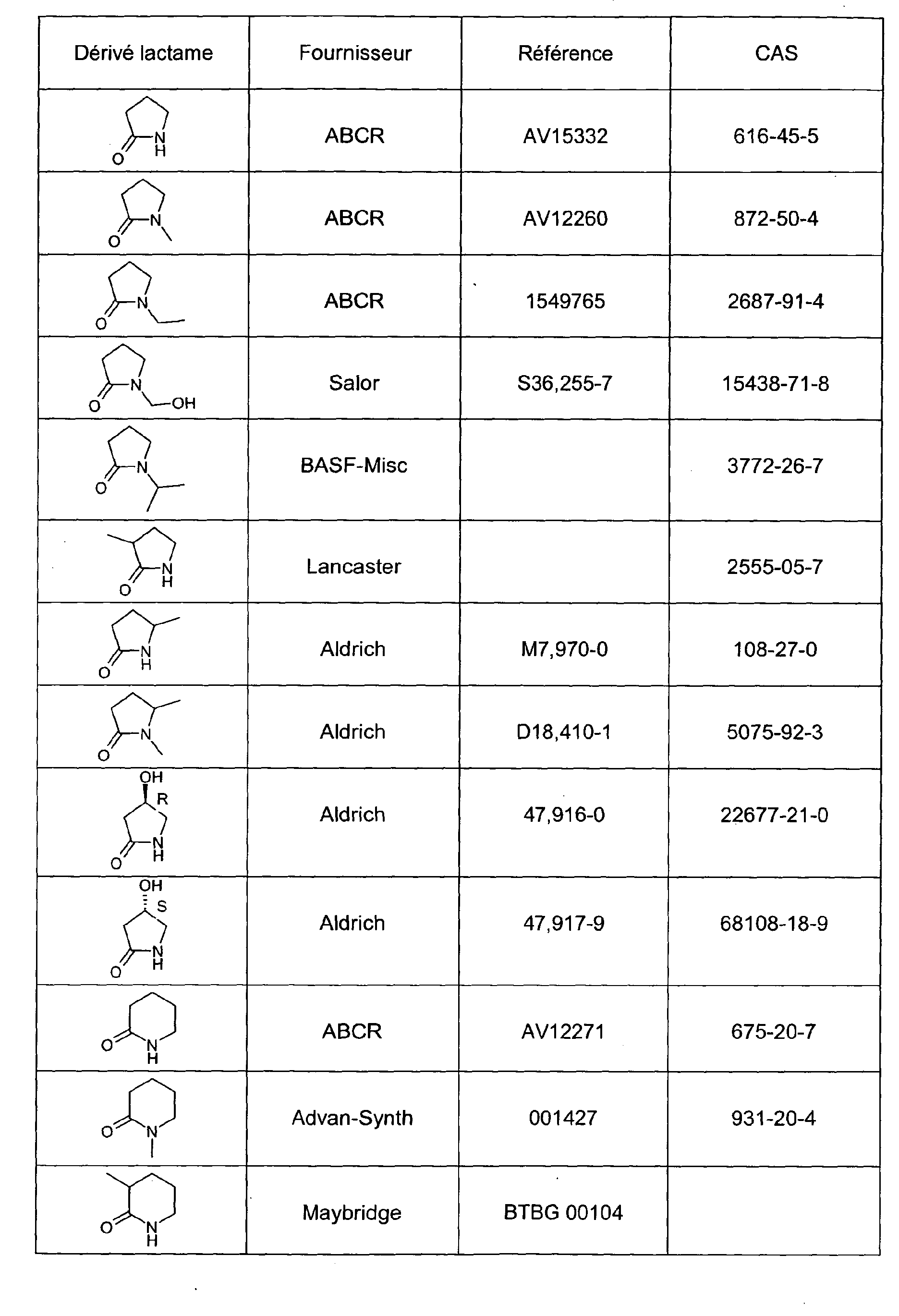

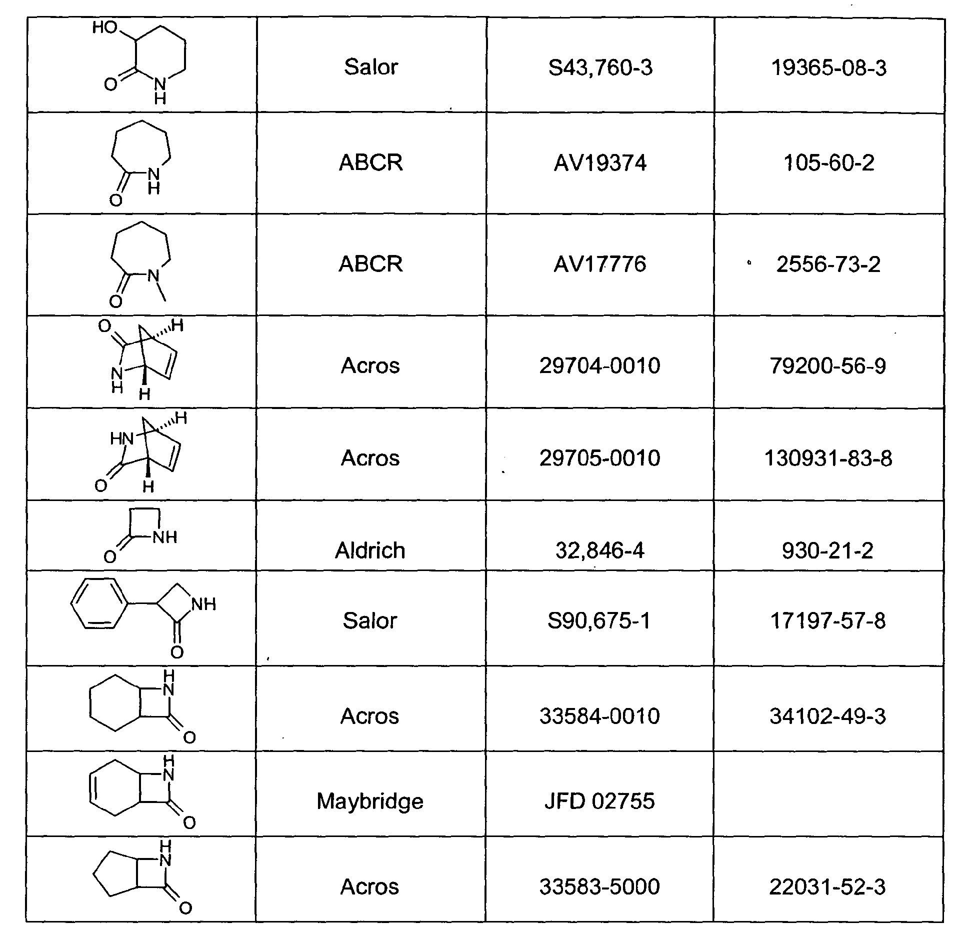

Les acétals de lactame peuvent être obtenus à partir des lactames selon le schéma réactionnel suivant cité dans les articles *Tetrahedron Letters* (1994), 35 (18), 2951 - 2954 et *Journal of Organic Chemistry* (1984), 49, 3659 - 3660 :

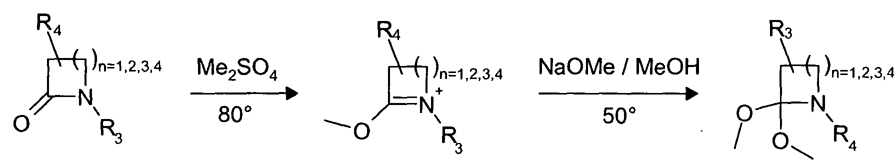

Selon le lactame de départ, des étapes de protection puis de déprotection de certains groupes fonctionnels comme OH ou NH, bien connues de l'homme de l'art, peuvent être nécessaires.

Parmi les composés de formule (1) préférentiels , on peut citer ceux de formule (II) ou (III) suivantes : 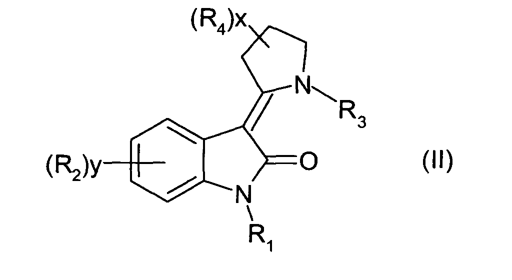 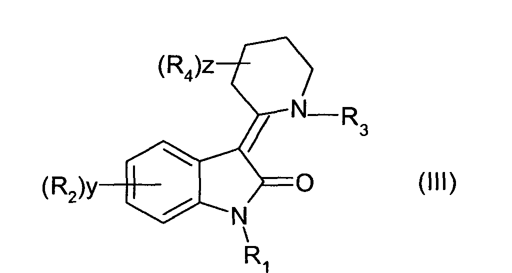 dans lesquelles :
R₁ et R₃, identiques ou différents, désignent l'hydrogène, un radical alkyle en C₁-C₈, linéaire ou ramifié, saturé ou insaturé, le groupe phényle ou un radical COR₅ ou SO₂R₅ avec R₅ désignant un radical alkyle en C₁-C₈ linéaire ou ramifié, saturé ou insaturé, ou le groupe phényle ;
R₂ désigne l'hydrogène ; un radical alkyle en C₁-C₈, linéaire ou ramifié, saturé ou insaturé ; le groupe phényle ; un des radicaux OR₅, NR₅R'₅, NR₅COR'₅, COOR₅ et
CONR₅R'₅ avec R₅ et R'₅, identiques ou différents, désignant l'hydrogène, un radical alkyle en C₁-C₈ linéaire ou ramifié, saturé ou insaturé, ou le groupe phényle ; le radical CF₃ ;
R₄ désigne l'hydrogène, un radical alkyle en C₁-C₈, linéaire ou ramifié, saturé ou insaturé, le groupe phényle ou un radical OR₅ avec R₅ désignant l'hydrogène, un radical alkyle en C₁-C₈ linéaire ou ramifié, saturé ou insaturé, ou le groupe phényle ;
x, y et z valent 1,2, 3 ou 4.

Parmi les dérivés de 3-(2-azacycloalkylidene)-1,3-dihydro-indol-2-one de formule (1) on citera encore plus particulièrement ceux de formule (II) où :
R₂ désigne l'hydrogène, OR₅, NR₅COR'₅ ou NR₅R'₅ avec R₅ et R'₅, identiques ou différents, désignant l'hydrogène, un radical alkyle en C₁-C₈ linéaire ou ramifié, saturé ou insaturé, ou le groupe phényle ;
R₄ désigne l'hydrogène ;
R₁ et R₃, identiques ou différents, désignent l'hydrogène, un radical alkyle en C₁-C₈, linéaire ou ramifié, saturé ou insaturé, ou le groupe phényle et encore plus particulièrement ceux décrits dans le tableau suivant, disponibles commercialement chez Specs/Biospecs :

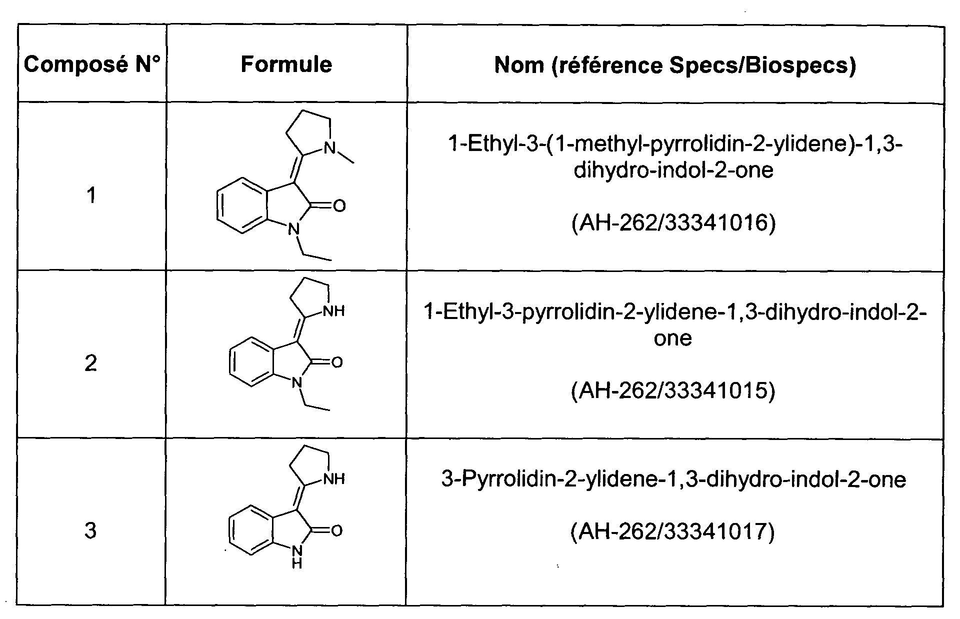

Les composés de formule (1) sont généralement présents dans la composition de l'invention dans des proportions comprises entre 0,01 % et 20 % en poids, de préférence entre 0,1 % et 10 % en poids, par rapport au poids total de la composition.

Les compositions conformes à l'invention peuvent comporter en plus d'autres filtres UV organiques ou inorganiques complémentaires actifs dans l'UVA et/ou l'UVB hydrosolubles ou liposolubles ou bien insolubles dans les solvants cosmétiques couramment utilisés.

Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres organiques complémentaires, on peut citer ceux désignés ci-dessous sous leur nom INCl :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés du dibenzoylméthane :

Butyl Methoxydibenzoylmethane vendu notamment sous le nom commercial « PARSOL 1789 » par HOFFMANN LA ROCHE,
Isopropyl Dibenzoylmethane,

### Dérivés cinnamiques:

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

### Dérivés de β,β'-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF, Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF, Benzophenone-12
2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK , Benzylidene Camphor Sulfonic Acid fabriqué sous le nom « MEXORYL SL» par CHIMEX, Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés de la triazine :

Anisotriazine vendu sous le nom commercial «TINOSORB S » par CIBA GEIGY,
Ethylhexyl triazone vendu notamment sous le nom commercial «UVINUL T150 » par BASF,
2,4,6-tris-(4'-amino benzalmalonate de diisobutyle)-s- triazine,
Diethylhexyl Butamido Triazone vendu sous le nom commercial « UVASORB HEB » par SIGMA 3V,

### Dérivés du phenyl benzotriazole :

Drometrizole Trisiloxane vendu sous le nom « Silatrizole » par RHODIA CHIMIE ,
Methylène -bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

Menthyl anthranilate vendu sous le nom commercial commercial « NEO HELIOPAN MA » par Haarmann et REIMER,

### Dérivés d'imidazolines :

Ethythexy) Dimethoxybenzylidene Dioxoimidazoline Propionate,

### Dérivés du benzalmalonate :

Polyorganosiloxane à fonction benzalmalonate comme le produit Polysilicone-15 vendu sous la dénomination commerciale « PARSOL SLX » par HOFFMANN LA ROCHE

### Dérivés de 4,4-diarylbutadiène :

- 1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom Uvasorb K2A par Sigma 3V ;
et leurs mélanges.

Les filtres UV organiques complémentaires préférentiels sont choisis parmi :
- Ethylhexyl Salicylate,
- Ethylhexyl Methoxycinnamate
- Octocrylene,
- Phenylbenzimidazole Sulfonic Acid,
- Benzophenone-3,
- Benzophenone-4,
- Benzophenone-5,
- 2-(4-diethylamino-2-hydroxybenzoyl)-benzoate de n-hexyle.
- 4-Methylbenzylidene camphor,
- Terephthalylidene Dicamphor Sulfonic Acid,
- Disodium Phenyl Dibenzimidazole Tetra-sulfonate,
- la 2,4,6-tris-(4'-amino benzalmalonate de diisobutyle)-s-triazine
- Anisotriazine,
- Ethylhexyl triazone,
- Diethylhexyl Butamido Triazone,
- Methylène bis-Benzotriazolyl Tetramethylbutylphénol
- Drometrizole Trisiloxane
- Polysilicone-15
- 1,1-dicarboxy (2,2'-diméthyl-propyl)-4,4-diphénylbutadiène
- 2,4-bis-[5-1(diméthylpropyl)benzoxazol-2-yl-(4-phenyl)-imino]-6-(2-ethylhexyl)-imino-1,3,5-triazine et leurs mélanges.

Les filtres complémentaires inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les filtres UV complémentaires conformes à l'invention sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions cosmétiques selon l'invention peuvent contenir en outre des agents de bronzage et/ou de brunissage artificiel de la peau (agents autobronzants) tels que la dihydroxyacétone (DHA).

Les compositions conformes à la présente invention peuvent comprendre en outre des adjuvants cosmétiques classiques notamment choisis parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C12-C15 vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Les épaississants peuvent être choisis notamment parmi les polymères acryliques réticulés comme les Carbomer, les polymères réticulés acrylates/C10-C30alkylacrylates du type Pemulen ou le polyacrylate-3 vendu sous le nom VISCOPHOBE DB 1000 par Amerchol ; les polyacrylamides tels que l'émulsion polyacrylamide, C13-C14 isoparraffine et laureth-7 vendue sous le nom SEPIGEL 305 par SEPPIC, les homopolymères ou copolymères d'AMPS tel l'HOSTACERIN AMPS vendu par CLARIANT, les gommes de guar et celluloses modifiées ou non telles que la gomme de guar hydroxypropylée, la méthylhydroxyéthylcellulose et l'hydroxypropylméthyl cellulose, la gomme de xanthane, les silices nanométriques de type Aerosil.

Bien entendu, l'homme de l'art veillera à choisir le ou les éventuels composés complémentaires cités ci-dessus et/ou leurs quantités de manière telle que les propriétés avantageuses attachées intrinsèquement aux composés conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, d'une huile, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Lorsque la composition cosmétique selon l'invention est utilisée pour le soin de l'épiderme humain, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, d'huile solaire, de bâtonnet solide, de poudre, de mousse aérosol ou de spray.

Lorsque la composition cosmétique selon l'invention est utilisée pour le soin des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des ongles, des lèvres, des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Un autre objet de l'invention est l'utilisation d'un dérivé de 3-(2-azacycloalkylidene)-1,3-dihydro-indol-2-one de formule (1) tel que définie ci-dessus dans une composition cosmétique ou dermatologique comme agent filtrant les radiations UV.

Un autre objet de l'invention est l'utilisation d'un dérivé de 3-(2-azacycloalkylidene)-1,3-dihydro-indol-2-one de formule (I) tel que définie ci-dessus dans une composition cosmétique comme agent de contrôle de la variation de la couleur de la peau due aux rayonnements UV.

Un autre objet de l'invention est l'utilisation d'un dérivé de 3-(2-azacycloalkylidene)-1,3-dihydro-indol-2-one de formule (1) tel que définie précédemment comme agent photostabilisant de polymères synthétiques tels que les matières plastiques ou de verres en particulier des verres de lunettes ou des lentilles de contact.

Des exemples concrets, mais nullement limitatifs, illustrant l'invention, vont maintenant être donnés.

### EXEMPLES

### EXEMPLE 1

### 1-Ethyl-3-(1-methyl-pyrrolidin-2-ylidene)-1,3-dihydro-indol-2-one (composé 1)

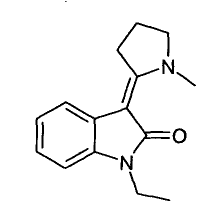

Ce composé peut être acheté chez Specs/Biospecs sous la référence AH-262/33341016 ou être obtenu selon le procédé de synthèse défini ci-dessus à partir de 1-Ethyl-1,3-dihydro-indol-2-one (cas : 61-28-9) et de 1-Méthyl-pyrrolidin-2-one (cas : 872-50-4).

Ce composé présente les propriétés de filtration solaire suivantes :
λₘₐₓ = 350 nm (92,8% acétonitrile + 7,2% DMSO).
εₘₐₓ = 17501 l.mol⁻¹.cm⁻¹.
E1% = 722.

### Ce composé est photostable comme le montre l'expérience suivante :

Après une heure d'irradiation au simulateur solaire (UVA = 18,6 mW/cm², UVB = 0,6 mW/cm²) d'un film huileux contenant 0,5 % de ce composé, le taux résiduel dudit composé est de 92%.

Les études de RPE (Résonance Paramagnétique Electronique) montrent que ce composé ne manifeste aucune activité pro-oxydante en milieu formulé sous irradiation dans les mêmes conditions.

### EXEMPLE 2

### 1-Ethyl-3-pyrrolidin-2-ylidene-1,3-dihydro-indol-2-one (composé 2)

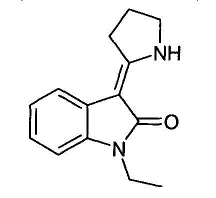

Ce composé peut être acheté chez Specs/Biospecs sous la référence AH-262/33341015 ou être obtenu selon le procédé de synthèse défini ci-dessus à partir de 1-Ethyl-1,3-dihydro-indol-2-one (cas : 61-28-9) et de 1-Pyrrolidin-2-one (cas : 616-45-5).

Ce composé présente les propriétés de filtration solaire suivantes :
λₘₐₓ = 326 nm (92,8% acétonitrile + 7,2% DMSO).
εₘₐₓ = 22600 l.mol⁻¹.cm⁻¹.

### EXEMPLE 3

### 3-Pyrrolidin-2-ylidene-1,3-dihydro-indol-2-one (composé 3)

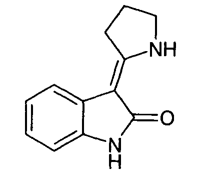

Ce composé peut être acheté chez Specs/Biospecs sous la référence AH-262/33341017 ou être obtenu selon le procédé de synthèse défini ci-dessus à partir de 1,3-Dihydroindol-2-one (cas : 59-48-3) et de 1-Pyrrolidin-2-one (cas : 616-45-5).

Ce composé présente les propriétés de filtration solaire suivantes :
λₘₐₓ = 328 nm (92,8% acétonitrile + 7,2% DMSO).
εₘₐₓ= 8100 l.mol⁻¹.cm⁻¹.

### EXEMPLE DE COMPOSITION 1

Comme exemple de composition, on peut citer une lotion solaire comprenant le composé de l'exemple 1 à la concentration de 0,5% dans le miglyol.

## Revendications

1. Composition cosmétique à usage topique, en particulier pour la photoprotection de la peau et/ou des cheveux, **caractérisée par le fait qu'**elle comprend, dans un support cosmétiquement acceptable au moins un dérivé de 3-(2-azacycloalkylidene)-1,3-dihydroindol-2-one répondant à la formule (1) suivante 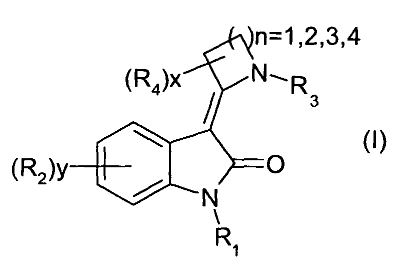 dans laquelle :
**R**_{**1**} **et R**_{**3**} sont identiques ou différents et peuvent être :
- un atome d'hydrogène,
- un groupe alkyle en C₁-C₂₂ linéaire ou ramifié, saturé ou insaturé, éventuellement substitué par un ou plusieurs groupes A₁,
- un cycle de 4 à 7 atomes, saturé ou insaturé, contenant éventuellement au moins un hétéroatome parmi N, O et S, éventuellement accolé à un autre cycle, éventuellement substitué par un ou plusieurs groupes A₁, ces cycles pouvant comporter une ou plusieurs fonctions carbonyle ou thiocarbonyle,
- un des groupes C(=NR₅)R'₅, C(=NR₅)NR'₅R"₅, COR₅, CSR₅, COOR₅, CONR₅R'₅, CSNR₅R'₅, SO₂R₅, SO₂NR₅R'₅ avec R₅, R'₅ et R"₅ identiques ou différents désignant l'hydrogène, un radical alkyle en C₁-C₂₂, linéaire ou ramifié ou un cycle de 4 à 7 atomes, pouvant contenir au moins un hétéroatome parmi N, O, S, isolé ou accolé à un autre cycle, ces cycles pouvant comporter une ou plusieurs fonctions carbonyle ou thiocarbonyle, le radical alkyle ou lesdits cycles étant saturés ou non et éventuellement substitués par au moins un substituant A₂.
**R**_{**2**} **et R**_{**4**} sont identiques ou différents et peuvent être :
- un atome d'hydrogène,
- un groupe alkyle en C₁-C₂₂ linéaire ou ramifié, saturé ou insaturé éventuellement, substitué par un ou plusieurs groupes A₁,
- un cycle de 4 à 7 atomes, saturé ou insaturé, contenant éventuellement au moins un hétéroatome parmi N, O et S, éventuellement substitué par un ou plusieurs groupes A₁, ce cycle pouvant accolé à un autre cycle ou aux cycles de la structure 3-(2-azacycloalkylidene)-1,3-dihydro-indol-2-one objet de l'invention, ces cycles pouvant comporter une ou plusieurs fonctions carbonyle ou thiocarbonyle, ce cycle pouvant également être chargé positivement comme imidazolium, pyridinium, pyrazolium, triazolium,
- un halogène comme F, Cl, Br,
- un des groupes CF₃, CN, OR₅, SR₅, NR₅R'₅, C(=NR₅)R'₅, C(=NR₅)NR'₅R"₅, NR₅C(=NR'₅)NR"₅R"'₅, COR₅, CSR₅, COOR₅, CONR₅R'₅, NR₅OR'₅, NR₅CONR'₅R"₅, CSNR₅R'₅, SO₂NR₅R'₅, NR₅SO₂R'₅, SO₂R₅, NR₅R'₅R"₅R"'₅⁺ avec R₅, R'₅, R"₅ et R"'₅ identiques ou différents désignant l'hydrogène, un radical alkyle en C₁-C₂₂, linéaire ou ramifié ou un cycle de 4 à 7 atomes, pouvant contenir au moins un hétéroatome parmi N, O, S, isolé ou accolé à un autre cycle, ces cycles pouvant comporter une ou plusieurs fonctions carbonyle ou thiocarbonyle, le radical alkyle ou lesdits cycles étant saturés ou non et éventuellement substitués par au moins un substituant A₂ ;
**A**_{**1**} peut être :
- un halogène comme F, CI, Br,
- un groupe alkyle en C₁-C₂₂ linéaire ou ramifié, saturé ou insaturé, éventuellement substitué par un ou plusieurs groupes A₂,
- un des groupes CF₃, CN, OR₅, SR₅, NR₅R'₅, C(=NR₅)R'₅, C(=NR₅)NR'₅R"₅, NR₅C(=NR'₅)NR"₅R"'₅, COR₅, CSR₅, COOR₅, CONR₅R'₅, NR₅COR'₅, NR₅CONR'₅R"₅, CSNR₅R'₅, SO₂NR₅R'₅, NR₅SO₂R'₅, SO₂R₅, SiR₅R'₅R"₅, SiR₅(OSiR'₅R"₅R"'₅)OSiR'₅R"₅R"'₅, NR₅R'₅R"₅R"'₅⁺ avec R₅, R'₅, R"₅ et R'"₅ identiques ou différents désignant l'hydrogène, un radical alkyle en C₁-C₂₂, linéaire ou ramifié ou un cycle de 4 à 7 atomes, pouvant contenir au moins un hétéroatome parmi N, O, S, isolé ou accolé à un autre cycle, ces cycles pouvant comporter une ou plusieurs fonctions carbonyle ou thiocarbonyle, le radical alkyle ou lesdits cycles étant saturés ou non et éventuellement substitués par au moins un substituant A₂
- un cycle de 4 à 7 atomes, saturé ou insaturé, contenant éventuellement au moins un hétéroatome parmi N, O et S, éventuellement substitué par un ou plusieurs groupes A₂, ce cycle pouvant accolé à un autre cycle ou aux cycles de la structure 3-(2-azacycloalkylidene)-1,3-dihydro-indol-2-one objet de l'invention, ces cycles pouvant comporter une ou plusieurs fonctions carbonyle ou thiocarbonyle, ce cycle pouvant également être chargé positivement comme imidazolium, pyridinium, pyrazolium, triazolium,
**A**_{**2**} peut étre :
- un halogène comme F, Cl, Br,
- un groupe alkyle en C₁-C₂₂ linéaire ou ramifié, saturé ou insaturé,
- un des groupes CF₃, CN, OR, SR, NRR', C(=NR)R', C(=NR)NR'R", NRC(=NR')NR"R"', COR, CSR, COOR, CONRR', NRCOR', NRCONR'R", CSNRR', SO₂NRR', NRSO₂R', SO₂R, SiRR'R", SiR(OSiR'R"R"')OSiR'R"R"', NRR'R"R"'⁺ avec R, R', R" et R"' identiques ou différents désignant l'hydrogène ou un radical alkyle en C₁-C₂₂, linéaire ou ramifié.
y vaut 1,2,3 ou 4 ;
x vaut de 1 à 2n+2.

2. Composition selon la revendication 1, où les composés de formule (I) sont choisis parmi ceux de formule (II) ou (III) suivantes : 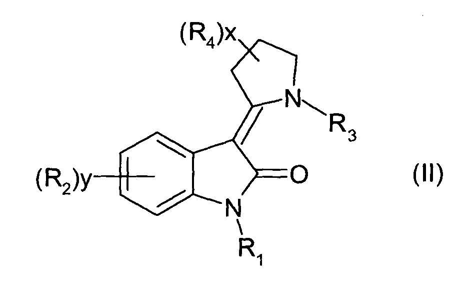 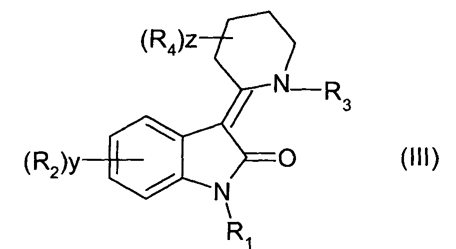 dans lesquelles :
R₁ et R₃, identiques ou différents, désignent l'hydrogène, un radical alkyle en C₁-C₈, linéaire ou ramifié, saturé ou insaturé, le groupe phényle ou un radical COR₅ ou SO₂R₅ avec R₅ désignant un radical alkyle en C₁-C₈ linéaire ou ramifié, saturé ou insaturé, ou le groupe phényle ;
R₂ désigne l'hydrogène ; un radical alkyle en C₁-C₈, linéaire ou ramifié, saturé ou insaturé ; le groupe phényle ; un des radicaux OR₅, NR₅R'₅, NR₅COR'₅, COOR₅ et
CONR₅R'₅ avec R₅ et R'₅, identiques ou différents, désignant l'hydrogène, un radical alkyle en C₁-C₈ linéaire ou ramifié, saturé ou insaturé, ou le groupe phényle ; le radical CF₃ ;
R₄ désigne l'hydrogène, un radical alkyle en C₁-C₈, linéaire ou ramifié, saturé ou insaturé, le groupe phényle ou un radical OR₅ avec R₅ désignant l'hydrogène, un radical alkyle en C₁-C₈ linéaire ou ramifié, saturé ou insaturé, ou le groupe phényle ;
x, y et z valent 1,2, 3 ou 4.

3. Composition selon la revendication 2, où les composés de formule (II) sont choisis parmi ceux pour lesquels :
R₂ désigne l'hydrogène, OR₅, NR₅COR'₅ ou NR₅R'₅ avec R₅ et R'₅, identiques ou différents, désignant l'hydrogène, un radical alkyle en C₁-C₈ linéaire ou ramifié, saturé ou insaturé, ou le groupe phényle ;
R₄ désigne l'hydrogène ;
R₁ et R₃, identiques ou différents, désignent l'hydrogène, un radical alkyle en C₁-C₈, linéaire ou ramifié, saturé ou insaturé, ou le groupe phényle.

4. Composition selon la revendication 3, où les composés de formule (II) sont choisis parmi les composés suivants :
1-Ethyl-3-(1-methyl-pyrrolidin-2-ylidene)-1,3-dihydro-indol-2-one
1-Ethyl-3-pyrrolidin-2-ylidene-1,3-dihydro-indol-2-one
Pyrrolidin-2-ylidene-1,3-dihydro-indol-2-one.

5. Composition selon l'une quelconque des revendications 1 à 4, **caractérisée par le fait que** le composé de formule (I) est présent dans la composition à une teneur allant de 0,01 à 20 % en poids, par rapport au poids total de la composition, de préférence allant de 0,1 % à 10 % en poids, par rapport au poids total de la composition.

6. Composition selon l'une quelconque des revendications 1 à 5, **caractérisée par le fait que** ledit support cosmétiquement acceptable se présente sous la forme d'une émulsion de type huile-dans-eau ou eau-dans-huile.

7. Composition selon l'une quelconque des revendications 1 à 5, **caractérisée par le fait qu'**elle comprend en outre un ou plusieurs filtres organiques ou inorganiques complémentaires actifs dans l'UV-A et/ou UV-B.

8. Composition selon la revendication 7, **caractérisée par le fait que** lesdits filtres organiques complémentaires sont choisis parmi les anthranilates ; les dérivés cinnamiques ; les dérivés de dibenzoylméthane ; les dérivés salicyliques, les dérivés du camphre ; les dérivés de triazine; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres; les dimères dérivés d'α-alkylstyrène; les 4,4-diarylbutadiènes et leurs mélanges.

9. Composition selon la revendication 7, **caractérisée par le fait que** lesdits filtres organiques complémentaires sont choisis parmi :
Ethylhexyl Salicylate,
Ethylhexyl Methoxycinnamate
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

10. Composition selon la revendication 7, **caractérisée par le fait que** les filtres complémentaires inorganiques sont des pigments ou des nanopigments d'oxydes métalliques, enrobés ou non.

11. Composition selon la revendication 10, **caractérisée par le fait que** lesdits pigments ou nanopigments sont choisis parmi les oxydes de titane, de zinc, de fer, de zirconium, de cérium et leurs mélanges, enrobés ou non.

12. Composition selon l'une quelconque des revendications 1 à 11, **caractérisée par le fait qu'**elle comprend en outre au moins un agent de bronzage et/ou de brunissage artificiel de la peau.

13. Composition selon l'une quelconque des revendications 1 à 12, **caractérisée par le fait qu'**elle comprend en outre au moins un adjuvant choisi parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants

14. Composition selon l'une quelconque des revendications 1 à 13, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une huile, d'une poudre, d'un bâtonnet solide, d'une mousse ou d'un spray.

15. Composition selon l'une quelconque des revendications 1 à 13, **caractérisée par le fait qu'**il s'agit d'une composition de maquillage des cils, des sourcils, des ongles ou de la peau et qu'elle se présente sous forme solide ou pâteuse, anhydre ou aqueuse, d'une émulsion, d'une suspension ou d'une dispersion.

16. Composition selon l'une quelconque des revendications 1 à 13, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

17. Utilisation d'un dérivé de 3-(2-azacycloalkylidene)-1,3-dihydro-indol-2-one de formule (1) tel que défini dans l'une quelconque des revendications précédentes dans une composition cosmétique ou dermatologique comme agent filtrant les radiations UV.

18. Utilisation d'un dérivé de 3-(2-azacycloalkylidene)-1,3-dihydro-indol-2-one de formule (1) tel que défini dans l'une quelconque des revendications précédentes dans une composition cosmétique comme agent de contrôle de la variation de la couleur de la peau due aux rayonnements UV.

19. Utilisation d'un dérivé de 3-(2-azacycloalkylidene)-1,3-dihydro-indol-2-one de formule (1) tel que défini dans l'une quelconque des revendications précédentes comme agent photostabilisant de polymères synthétiques ou de verres.
